# EUROPÄISCHE PATENTSCHRIFT

(11) **EP 3 718 980 B1**
(45) Veröffentlichungstag und Bekanntmachung des Hinweises auf die Patenterteilung: **03.08.2022**
(21) Anmeldenummer: 19167345.8
(22) Anmeldetag: 04.04.2019
(51) Int. Cl.: C03C 3/097, C03C 4/00, C03C 4/02, C03C 8/08, C03C 8/14, C03C 10/00

(54) **VERFAHREN ZUR HERSTELLUNG VON MEHRFARBIGEN GLASKERAMIK-ROHLINGEN**
METHOD FOR THE PREPARATION OF MULTICOLOUR GLASS CERAMIC BLANKS
PROCÉDÉ DE FABRICATION D'ÉBAUCHES EN CÉRAMIQUE MULTICOLORE

(43) Veröffentlichungstag der Anmeldung: 07.10.2020
(73) Patentinhaber: Ivoclar Vivadent AG, 9494 Schaan (LI)
(72) Erfinder: Arnold, Lars, 9473 Gams (CH); Bürke, Harald, 6820 Frastanz (AT); Engels, Alexander, 6800 Feldkirch (AT)
(74) Vertreter: Uexküll & Stolberg

(56) Entgegenhaltungen:
- EP-A1- 3 168 199
- EP-A1- 3 453 686
- EP-A1- 3 696 149
- WO-A1-2013/167722
- DE-A1- 10 336 913

## Beschreibung

Die Erfindung betrifft ein Verfahren, mit dem in einfacher Weise mehrfarbige Glaskeramik-Rohlinge erzeugt werden können, die die optischen Eigenschaften von natürlichem Zahnmaterial sehr gut nachahmen können und sich insbesondere für die einfache Herstellung von ästhetisch anspruchsvollen Dentalrestaurationen mit sehr guten optischen und mechanischen Eigenschaften eignen.

Es stellt eine große Herausforderung dar, Rohlinge zu schaffen, die den vielfältigen Anforderungen an einen Einsatz im Bereich der Dentaltechnik gerecht werden. Derartige Rohlinge sollen nicht nur einfach herstellbar sein, sondern sie sollen auch einfach zu den gewünschten Dentalrestaurationen formbar sein und trotzdem hochfeste Restaurationen ergeben. Schließlich sollen bereits die Rohlinge einen derartigen Aufbau haben, dass die aus ihnen hergestellten Restaurationen optische Eigenschaften aufweisen, die denen des natürlichen Zahnmaterials sehr nahekommen, damit eine aufwendige nachträglich Verblendung der Restaurationen entfallen kann. Denn natürliche Zähne sind nicht einfarbig, sondern sie haben eine komplexe Farbgebung, indem verschiedene Bereiche desselben Zahnes sich im Allgemeinen in ihrer Farbe und in ihrer Transluzenz voneinander unterscheiden.

Rohlinge für den Einsatz in der Dentaltechnik sind aus dem Stand der Technik bekannt.

Die DE 103 36 913 A1 beschreibt Rohlinge auf Basis von Lithiummetasilikat-Glaskeramik, die durch Wärmebehandlung von Glas-Rohlingen aus gegossenem Ausgangsglas, sogenannten Massivglas-Rohlingen oder monolithischen Glas-Rohlingen, hergestellt werden. Diese Verfahrensweise wird daher auch als "Massivglas-Technologie" bezeichnet. Die hergestellten Rohlinge können aufgrund ihrer relativ geringen Festigkeit in einfacher Weise maschinell bearbeitet und durch weitere Wärmebehandlung in hochfeste Dentalrestaurationen auf Basis von Lithiumdisilikat-Glaskeramik umgewandelt werden. Bei den hergestellten Rohlingen handelt es sich aber lediglich um einfarbige Rohlinge, die demnach auch nur zu einfarbigen Dentalrestaurationen führen. Zur Erzeugung von Mehrfarbigkeit ist daher auch eine aufwendige nachträgliche Verblendung der hergestellten Dentalrestaurationen erforderlich.

H. Zhang et al. beschreiben in J. Am. Ceram. Soc. 98; 3659-3662 (2015) die Herstellung einer Lithiummetasilikat-Glaskeramik durch Heißpressen eines speziellen Glaspulvers bei 760°C für 30 min unter Verwendung eines Drucks von 30 MPa. Dabei findet offenbar vornehmlich Oberflächenkristallisation statt, was wahrscheinlich ein Grund für die geringe Biegefestigkeit der aus dieser Glaskeramik durch weitere Wärmebehandlung bei 855° erhaltenen Lithiumdisilikat-Glaskeramik ist.

Die WO 2014/124879 beschreibt mehrfarbige Lithiumsilikat-Rohlinge, die unterschiedlich gefärbte monolithische Schichten aufweisen. Zu ihrer Herstellung werden Schichten aus unterschiedlich gefärbtem Massivglas miteinander verbunden, z.B. indem auf eine vorhandene Schicht aus Massivglas die Schmelze eines anders eingefärbten Glases gegossen wird, und anschließend eine Wärmebehandlung erfolgt. Zur guten Anpassung an die optischen Eigenschaften von zu ersetzendem natürlichen Zahnmaterial ist es allerdings erforderlich, eine ganze Reihe von unterschiedlich gefärbten Schichten aus Massivglas vorzusehen, was mithilfe der beschriebenen Verfahrensweisen sehr aufwendig und langwierig ist. Überdies ist das Imitieren eines kontinuierlichen Farbverlaufs auf diese Weise nicht möglich.

Erfindungsgemäß sollen die beschriebenen Probleme bei den konventionellen Verfahren vermieden werden. Der Erfindung liegt insbesondere die Aufgabe zugrunde, ein Verfahren zur Verfügung zu stellen, mit dem in einfacher Weise mehrfarbige Glaskeramik-Rohlinge hergestellt werden können, mit denen die optischen Eigenschaften von natürlichem Zahnmaterial sehr gut nachgeahmt werden können, denen in einfacher Weise maschinell die Form der schließlich gewünschten Dentalrestauration gegeben werden kann und die nach der Formgebung zu Dentalrestaurationen mit ausgezeichneten mechanischen und optischen Eigenschaften umgewandelt werden können.

Diese Aufgabe wird durch das Verfahren nach den Ansprüchen 1 bis 15 gelöst. Gegenstand der Erfindung ist ebenfalls der mehrfarbige Glaskeramik-Rohling nach Anspruch 16, die Verwendung des Glaskeramik-Rohlings nach Anspruch 17 sowie das Verfahren zur Herstellung einer Dentalrestauration nach den Ansprüchen 18 bis 21.

Das erfindungsgemäße Verfahren zur Herstellung eines mehrfarbigen Glaskeramik-Rohlings für dentale Zwecke mit Lithiumsilikat als Hauptkristallphase, zeichnet sich dadurch aus, dass
(a) (i) unterschiedlich gefärbte Pulver von Lithiumsilikat-Gläsern oder (ii) Suspensionen von unterschiedlich gefärbten Pulvern von Lithiumsilikat-Gläsern in flüssigen Medien in eine Form eingebracht werden, um einen Glas-Rohling zu bilden,
(b) gegebenenfalls der Glas-Rohling aus Schritt (a) durch Pressen verdichtet wird,
(c) der Glasrohling aus Schritt (a) oder (b) wärmebehandelt wird, um einen Glaskeramik-Rohling mit Lithiumsilikat als Hauptkristallphase zu erhalten und
(d) der Glaskeramik-Rohling aus Schritt (c) durch Heißpressen verdichtet wird.

Überraschenderweise gestattet das erfindungsgemäße Verfahren die Erzeugung eines Glaskeramik-Rohlings, dem in sehr einfacher Weise Mehrfarbigkeit verliehen werden kann und der die Herstellung von dentalen Restaurationen gestattet, die nicht nur die optischen Eigenschaften von natürlichem Zahnmaterial simulieren und insbesondere kontinuierliche Farbverläufe aufweisen können, sondern die gleichzeitig auch über ausgezeichnete mechanische Eigenschaften verfügen.

Die Mehrfarbigkeit des erfindungsgemäß hergestellten Glaskeramik-Rohlings bedeutet, dass er Bereiche mit unterschiedlicher Zusammensetzung hat, die bei Umwandlung des Rohlings zur gewünschten Dentalrestauration mittels Wärmebehandlung zu Bereichen mit unterschiedlicher Farbe führen und somit der Dentalrestauration Mehrfarbigkeit verleihen. Dabei sind mit Unterschieden in der Farbe auch Unterschiede in der Transluzenz, Opaleszenz und/oder Fluoreszenz gemeint.

Die Farbe kann insbesondere über den Lab-Wert oder mit Hilfe eines in der Dentalindustrie üblichen Farbschlüssels bestimmt werden.

Die Transluzenz kann insbesondere über den Kontrastwert (CR-Wert) gemäß British Standard BS 5612 bestimmt werden.

Die Opaleszenz kann mithilfe photometrischer Messung, insbesondere in der in der WO 2014/209626 beschriebenen Weise, bestimmt werden.

Die Fluoreszenz kann insbesondere mithilfe von Fluoreszenzspektrometern, z.B. einem Fluoreszenz-Spektrometers vom Typ FL1039 unter Verwendung eines Photomultiplier-Detektors vom Typ PMT 1424M, beide von Horiba Jobin Yvon GmbH, bestimmt werden.

In Schritt (a) des erfindungsgemäßen Verfahrens werden in einer ersten Variante (i) unterschiedlich gefärbte Pulver von Lithiumsilikat-Gläsern oder in einer zweiten Variante (ii) Suspensionen von unterschiedlich gefärbten Pulvern von Lithiumsilikat-Gläsern in flüssigen Medien in eine Form eingebracht, um einen Glas-Rohling zu bilden.

Dabei sind mit unterschiedlich gefärbten Pulvern bei beiden Varianten (i) und (ii) Pulver mit unterschiedlichen Zusammensetzungen gemeint, die bei Weiterverarbeitung über den erfindungsgemäß hergestellten Glaskeramik-Rohling zur Dentalrestauration Bereiche mit unterschiedlicher Farbe in der Dentalrestauration erzeugen. Diese gewünschte Mehrfarbigkeit der Dentalrestauration kann insbesondere eine sich kontinuierlich verändernde Farbe, wie ein Farb-Verlauf und/oder Transluzenz-Verlauf, sein. Derartige Verläufe von Farbe und Transluzenz treten regelmäßig beim natürlichen Zahnmaterial, z.B. zwischen Zahnbein und Zahnschneide, auf.

Die unterschiedliche Färbung der bei beiden Varianten (i) und (ii) eingesetzten Pulver von Lithiumsilikat-Gläsern kann dabei durch die unterschiedliche Zusammensetzung der Lithiumsilikat-Gläser oder auch durch Zumischung von Additiven, wie Farbkomponenten und/oder Fluoreszenzkomponenten, zu den Gläsern, erzeugt werden. Das stellt einen besonderen Vorteil des erfindungsgemäßen Verfahrens dar, da auf diese Weise eine unterschiedliche Farbgebung der Pulver nicht nur durch Komponenten der Gläser, wie färbende Ionen, sondern auch durch die Zugabe von Pigmenten zu den Gläsern, wie Farbpigmenten und/oder Fluoreszenzpigmenten, erzielt werden kann. Bei der Verwendung der sogenannten Massivglas-Technologie, d. h. der Verwendung von gegossenen monolithischen Glasrohlingen, ist demgegenüber eine Einfärbung nur durch Ionenfärbung möglich.

Zur Herstellung der Lithiumsilikat-Gläser wird üblicherweise zunächst eine Mischung von geeigneten Ausgangsmaterialien, wie z.B. Carbonaten, Oxiden, Phosphaten und Fluoriden, bei Temperaturen von insbesondere 1300 bis 1600°C für 1 bis 10 h erschmolzen. Die erhaltene Glasschmelze wird dann in Wasser gegossen, um eine Glasfritte zu erzeugen. Zur Erzielung einer besonders hohen Homogenität kann die Glasfritte erneut aufgeschmolzen und die erhaltene Glasschmelze durch Eingießen in Wasser erneut zu einer Glasfritte umgewandelt werden. Schließlich wird die Glasfritte zu Pulver mit der gewünschten Teilchengröße zerkleinert. Hierfür geeignete Mühlen sind zum Beispiel Walzenmühlen, Kugelmühlen oder Gegenstrahlmühlen. Den erhaltenen Pulvern können dann noch Additive zugegeben werden, um die unterschiedlichen Pulver für die erste Variante (i) und die zweite Variante (ii) zu ergeben.

Die Pulver der ersten Variante (i) können als Additive zum Beispiel Farb- und/oder Fluoreszenzpigmente, wie zum Beispiel keramische Farbkörper, Presshilfsmittel und insbesondere Bindemittel enthalten. Dabei dienen die Bindemittel dem Zusammenhalt der Pulverteilchen und sie fördern daher den Erhalt eines stabilen Glas-Rohlings. Bevorzugte Beispiele von Bindemitteln sind Polyvinylalkohole und Cellulosederivate, wie Natriumcaboxymethylcellulose. Als Presshilfsmittel werden bevorzugt Polyethylenglykole oder Stearate verwendet.

Üblicherweise enthalten die Pulver der ersten Variante (i) neben dem Lithiumsilikat-Glas bis zu 10 Gew.-% Additive. Dabei werden Farb- und/oder Fluoreszenzpigmente typischerweise in einer Menge von 0 bis 5 Gew.-%, Presshilfsmittel typischerweise in einer Menge von 0 bis 3 Gew.-%, bevorzugt 0 bis 1 Gew.-%, und Bindemittel typischerweise in einer Menge von 0 bis 5 Gew.-%, bevorzugt 0,3 bis 3 Gew.-%, als bevorzugte Additive eingesetzt.

Die Pulver der in der zweiten Variante (ii) eingesetzten Suspensionen können als Additive Farb- und/oder Fluoreszenzpigmente, enthalten, wie sie zuvor von ihrer Art und Menge für die Variante (i) angegebenen sind.

Zur Herstellung der Suspensionen werden diese Pulver üblicherweise in flüssigen Medien und insbesondere wässrigen Medien suspendiert. Die flüssigen Medien enthalten bevorzugt Hilfsmittel, wie Bindemittel, Dispergiermittel, insbesondere in einer Menge von 0 bis 3 Gew.-%, Mittel zum Einstellen der Viskosität, insbesondere in einer Menge von 0 bis 3 Gew.-%, und Mittel zum Einstellen des pH-Wertes, insbesondere in einer Menge von 0 bis 1 Gew.-%, bevorzugt 0,001 bis 0,5 Gew.-%.

Bevorzugte Bindemittel sind Polyvinylalkohole und Cellulosederivate, wie Natriumcaboxymethylcellulose. Beispiele für geeignete Dispergiermittel sind Polymere und Lecithine. Beispiele für geeignete Mittel zum Einstellen der Viskosität sind Xanthane und Stärken. Beispiele für geeignete Mittel zum Einstellen des pH-Wertes sind anorganische oder organische Säuren, wie Essigsäure und Salzsäure.

Die Suspensionen enthalten insbesondere 30 bis 90 Gew.-% und bevorzugt 40 bis 70 Gew.-% Pulver.

Es werden in Schritt (a) des erfindungsgemäßen Verfahrens mindestens zwei unterschiedlich gefärbte Pulver (i) oder mindestens zwei Suspensionen unterschiedlich gefärbter Pulver (ii) eingesetzt.

Die unterschiedlichen Pulver (i) oder die unterschiedlichen Suspensionen (ii) werden in geeigneter Weise in die Form eingebracht, um die gewünschte Mehrfarbigkeit in dem Glaskeramik-Rohling und der daraus hergestellten Dentalrestauration und insbesondere einen gewünschten Verlauf von Farbe, Transluzenz und/oder Fluoreszenz zu erzeugen. Dies wird üblicherweise dadurch erreicht, dass die unterschiedlichen Pulver (i) oder die unterschiedlichen Suspensionen (ii) in gesteuerter Weise in die Form eingebracht werden. Beispielsweise kann durch geeignet gesteuertes Mischen von entweder unterschiedlichen Pulvern oder unterschiedlichen Suspensionen eine kontinuierliche Änderung der Zusammensetzung der in die Form eingebrachten Mischung erreicht und damit ein kontinuierlicher Farbverlauf erzeugt werden. Beispielsweise können zwei oder mehr unterschiedliche Pulver derart in die Form eingebracht werden, dass zunächst nur das erste Pulver zugegeben wird, dem nach und nach ein stetig höherer Anteil an mindestens einem weiteren Pulver zugemischt wird.

In einer bevorzugten Ausführungsform des erfindungsgemäßen Verfahrens werden die Pulver (i) oder Suspensionen (ii) in der Weise in die Form eingebracht, dass der hergestellte mehrfarbige Glaskeramik-Rohling eine sich kontinuierlich verändernde Farbe hat. Mit einem derartigen Glaskeramik-Rohling lässt sich der Farbverlauf von natürlichem Zahnmaterial besonders gut simulieren.

In einer bevorzugten Ausführungsform des erfindungsgemäßen Verfahrens werden in Schritt (a) unterschiedliche Pulver (i) in die Form eingebracht und der optionale Schritt (b) wird durchgeführt. Das Pressen gemäß Schritt (b) führt dazu, dass der Glas-Rohling eine gute Festigkeit hat. Der gepresste Glas-Rohling wird auch als Pulverpressling bezeichnet, da er aus gepressten Pulverteilchen besteht.

In einer anderen bevorzugten Ausführungsform des erfindungsgemäßen Verfahrens werden in Schritt (a) unterschiedliche Suspensionen (ii) in die Form eingebracht und die flüssigen Medien werden entfernt.

Der nach Entfernung der flüssigen Medien erhaltene Glas-Rohling wird in der Regel noch einem Trocknungsvorgang bei 10 bis 100°C unterworfen. Es ist ein besonderer Vorteil dieser Ausführungsform, dass der danach erhaltene Glas-Rohling auch ohne eine weitere Verdichtung durch Pressen regelmäßig über eine ausreichende Festigkeit für die Weiterverarbeitung verfügt.

Die Suspensionen (ii) werden üblicherweise in eine Form eingebracht, die über Poren verfügt. Das Einbringen erfolgt dabei üblicherweise durch Eingießen. Bei den Poren handelt es sich um Ausnehmungen, durch die das flüssige Medium aus den Suspensionen zumindest teilweise entfernt werden kann, sodass sich die Pulver-Teilchen in der Form abscheiden und schließlich den Glas-Rohling bilden können.

Typischerweise wird im Wesentlichen das gesamte flüssige Medium über die Poren entfernt. Es ist allerdings auch möglich, dass verbleibende nicht über die Poren entfernte Flüssigkeit aus der Form abgegossen oder abgesaugt wird. Das wird üblicherweise dann der Fall sein, wenn sich bereits eine ausreichend dicke Schicht von Pulver-Teilchen abgeschieden hat.

Bei der Form kann es sich beispielsweise um eine der üblicherweise für Schlickerguss- oder Druckgussverfahren eingesetzten Formen handeln. Dies sind insbesondere Formen mit einer Wandung aus Gips, durch die aufgrund der Kapillarwirkung der Gipsporen der Suspension flüssiges Medium, wie Wasser, entzogen werden kann. Es sind aber auch Formen aus Kunststoff, Keramik oder Metall einsetzbar, die bereits über Poren verfügen oder bei denen Poren vorgesehen werden, indem sie z.B. mit Filterelementen, wie Membran-, Papier- und Sinterfiltern, versehen werden.

Die eingesetzte Form ist insbesondere mehrteilig, um die einfache Entfernung des gebildeten Rohlings aus der Form zu erleichtern. In einer besonders bevorzugten Ausführungsform weist die Form Anschlüsse auf, über die auf die eingebrachte Suspension Druck, zum Beispiel mittels Druckluft, ausgeübt und/oder ein Unterdruck an die Poren angelegt werden kann. Beide Maßnahmen dienen dazu, die Entfernung des flüssigen Mediums aus der Form zu beschleunigen und damit das Verfahren abzukürzen. Mit ihrer Hilfe ist eine sehr schnelle und damit ökonomische Herstellung von Glas-Rohlingen möglich, was insbesondere bei einer Fertigung in industriellem Maßstab von besonderem Vorteil ist.

Die Entfernung der flüssigen Medien kann überdies auch durch Gefriertrocknung erfolgen. Dazu wird der durch Schlickerguss erzeugte Glas-Rohling in einer dichten, aber flexiblen Form, zum Beispiel aus Silikon, auf Temperaturen abgekühlt, bei denen die flüssigen Komponenten der Suspension gefrieren. Durch anschließende Sublimation dieser flüssigen Komponenten bei vermindertem Druck erfolgt deren Entfernung und damit die vollständige Trocknung. Eine separate Wärmebehandlung zur Trocknung des Rohlings ist dann regelmäßig nicht mehr erforderlich.

In einer weiteren bevorzugten Ausführungsform des erfindungsgemäßen Verfahrens haben die Pulver (i) eine Teilchengröße von 0,5 bis 150 µm, insbesondere 1 bis 100 µm, und die Pulver der Suspensionen (ii) eine Teilchengröße von 0,5 bis 80 µm, insbesondere 0,5 bis 70 µm, gemessen durch Laserbeugung gemäß ISO 13320 (2009). Dabei wurden die zur Bestimmung der Teilchengröße verwendeten Proben insbesondere gemäß DIN ISO 14887 (2010) hergestellt, wobei Wasser als Lösungsmittel verwendet wurde, um die Proben zu dispergieren.

Die mittlere Teilchengröße als d₅₀-Wert der Pulver (i) und (ii) beträgt 5 bis 30 µm, bevorzugt 10 bis 20 µm, bestimmt auf der Basis der durch Laserbeugung gemäß ISO 13320 (2009) gemessenen Volumenanteile.

Der in Schritt (a) gebildete Glas-Rohling hat üblicherweise die Form eines Blocks, Zylinders oder einer Scheibe, da Rohlingen derartiger Geometrie in üblichen Bearbeitungsmaschinen leicht die Form der gewünschten Dentalrestauration gegeben werden kann. Der Rohling kann auch bereits eine einstückig mit dem Rohling ausgebildete Haltevorrichtung, wie einen Haltezapfen, aufweisen, was dessen späteres Anbringen zum Beispiel durch Ankleben überflüssig macht.

Demnach hat auch die eingesetzte Form üblicherweise eine Geometrie, die die Erzeugung solcher Rohlinge gestattet. Die Form kann einteilig oder zur leichteren Entfernung des erzeugten Rohlings auch mehrteilig, insbesondere 3-teilig, sein.

In dem optionalen Schritt (b) des erfindungsgemäßen Verfahrens wird der Glas-Rohling aus Schritt (a) durch Pressen verdichtet. Es ist bevorzugt, dass die in Schritt (a) eingesetzten Pulver der ersten Variante (i) diesem optionalen Schritt unterworfen werden.

Es ist weiter bevorzugt, dass das Pressen in Schritt (b) bei einer Temperatur von weniger als 60°C, bevorzugt bei 15 bis 35°C, und insbesondere bei einem Druck von 20 bis 120 MPa, bevorzugt 50 bis 120 MPa, erfolgt. Regelmäßig findet das Pressen bei Raumtemperatur statt, und es wird daher auch als Kaltpressen bezeichnet.

In Schritt (c) des erfindungsgemäßen Verfahrens wird der Glasrohling aus Schritt (a) und (b) wärmebehandelt, um einen Glaskeramik-Rohling mit Lithiumsilikat als Hauptkristallphase zu erhalten. Dabei ist es bevorzugt, die Wärmebehandlung bei einer Temperatur von weniger als 700°C, bevorzugt 550 bis 690°C, und für eine Dauer von insbesondere 2 bis 60 min, bevorzugt 5 bis 30 min, durchzuführen.

Üblicherweise wird der Glasrohling vor der Wärmebehandlung bei Temperaturen von insbesondere 400 bis 450°C entbindert, um eventuell vorhandene Bindemittel oder andere organische Additive zu entfernen. Danach wird der Glasrohling regelmäßig direkt auf die Temperatur der Wärmebehandlung aufgewärmt. Das Aufheizen auf die Temperatur der Wärmebehandlung und das Halten dieser Temperatur bewirken die Bildung von Keimen und die Kristallisation von Lithiumsilikat, insbesondere von Lithiummetasilikat, als Hauptkristallphase.

In Schritt (d) des erfindungsgemäßen Verfahrens wird der Glaskeramik-Rohling aus Schritt (c) durch Heißpressen verdichtet. Durch diese Verdichtung gelingt es überraschenderweise, dem Glaskeramik-Rohling eine Dichte zu verleihen, die sich von der Dichte eines auf herkömmliche Weise durch die Massivglas-Technologie erzeugten Glaskeramik-Rohlings nicht wesentlich unterscheidet. Während bei dem erfindungsgemäßen Verfahren Glaspulver und damit die sogenannte "Pulver-Technologie" zur Erzeugung des Glaskeramik-Rohlings eingesetzt werden, wird bei der Massivglas-Technologie Glasschmelze in eine Form gegossen, um einen monolithischen Glasrohling zu bilden, der nach Wärmebehandlung den gewünschten Glaskeramik-Rohling ergibt.

Das Heißpressen in Schritt (d) wird vorzugsweise in einer Form durchgeführt, an der Glas nicht anhaftet. Geeignete Werkstoffe für eine solche Form sind Werkstoffe auf Kohlenstoffbasis und Keramiken auf Basis von Nitriden. Metallische Werkstoffe sind ebenfalls geeignet, wenn ein geeignetes Trennmittel zwischen Form und zu verdichtendem Glaskeramik-Rohling gebracht wird.

Es ist bevorzugt, dass das Heißpressen bei einer Temperatur von 650 bis 780°C, insbesondere 700 bis 750°C, und bei einem Druck von insbesondere 5 bis 50 MPa, bevorzugt 10 bis 30 MPa, erfolgt.

Es ist weiter bevorzugt, dass das Heißpressen für eine Dauer von 0,1 bis 10 min, bevorzugt 0,3 bis 5 min, erfolgt. Es ist ein weiterer Vorteil des erfindungsgemäßen Verfahrens, dass ein Heißpressen für eine so kurze Dauer ausreichend ist, um einen Glaskeramik-Rohling zu erzeugen, aus dem in schneller und einfacher Weise Dentalrestaurationen mit ausgezeichneten optischen und mechanischen Eigenschaften hergestellt werden können.

In einer weiteren bevorzugten Ausführungsform erfolgt das Heißpressen bei einem Atmosphärendruck von weniger als 0,1 bar und bevorzugt bei 0,01 bis 0,08 bar.

Der nach dem Heißpressen erhaltene mehrfarbige Glaskeramik-Rohling hat bevorzugt eine Dichte von 2,4 bis 2,6 und insbesondere 2,44 bis 2,56 g/cm³. Die Bestimmung der Dichte dieses Rohlings erfolgte nach Archimedes in deionisiertem Wasser. Damit hat der erfindungsgemäße Glaskeramik-Rohling überraschenderweise eine ähnliche Dichte wie ein entsprechender durch Massivglas-Technologie erzeugter konventioneller Rohling.

Bei den nach den Schritten (a), (b) und (c) erhaltenen Glas-Rohlingen und Glaskeramik-Rohlingen erfolgte die Bestimmung der Masse durch Wägung und die des Volumens durch optische Vermessung mittels Streifenprojektionsmethode (3D-Scanner ATOS der Firma GOM GmbH, Deutschland. Die Dichte wurde dann nach der Formel ρ = Masse / Volumen berechnet.

Der erhaltene mehrfarbige Glaskeramik-Rohling weist insbesondere Lithiummetasilikat als Hauptkristallphase auf. Es ist weiter bevorzugt, dass der Glaskeramik-Rohling weniger als 20 Gew.-%, insbesondere weniger als 10 Gew.-%, bevorzugt weniger als 5 Gew.-% und ganz besonders bevorzugt weniger als 3 Gew.-% Lithiumdisilikat aufweist, da höhere Mengen an Lithiumdisilikat-Kristallen die Formgebung mittels maschineller Bearbeitung beeinträchtigen können.

Mit dem Begriff "Hauptkristallphase" wird die Kristallphase bezeichnet, die von allen in der Glaskeramik vorhandenen Kristallphasen den höchsten Massenanteil hat. Die Bestimmung der Massen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al₂O₃-SiO₂ mit ZrO₂ als Keimbildner", Universität Jena 2011, beschrieben.

In einer bevorzugten Ausführungsform enthält der Glaskeramik-Rohling mehr als 10 Gew.-%, bevorzugt mehr als 20 Gew.-% und besonders bevorzugt mehr als 25 Gew.-% an Lithiummetasilikat-Kristallen.

Der mehrfarbige Glaskeramik-Rohling enthält insbesondere mindestens eine und vorzugsweise alle der folgenden Komponenten in den angegebenen Mengen:

| Komponente | | Gew.-% |
|---|---|---|
| | | |
| SiO₂ | 64,0 bis 75,0, bevorzugt 64,0 bis 72,0 | |
| Li₂O | 13,0 bis 17,0, bevorzugt 13,5 bis 16,0 | |
| K₂O | | 0 bis 5,0, bevorzugt 3,0 bis 5,0 |
| Al₂O₃ | | 0,5 bis 5,0, bevorzugt 1,5 bis 3,5 |
| P₂O₅ | | 2,0 bis 5,0, bevorzugt 2,5 bis 4,0 |

In einer weiter bevorzugten Ausführungsform enthält der mehrfarbige Glaskeramik-Rohling mindestens eine und vorzugsweise alle der folgenden Komponenten in den angegebenen Mengen:

| Komponente | | Gew.-% |
|---|---|---|
| | | |
| SiO₂ | 64,0 bis 75,0 | |
| Li₂O | 13,0 bis 17,0 | |
| K₂O | | 0 bis 5,0 |
| Al₂O₃ | | 0,5 bis 5,0 |
| P₂O₅ | | 2,0 bis 5,0 |
| ZrO₂ | | 0 bis 5,0 |
| MgO | | 0 bis 5,0 |
| SrO | | 0 bis 5,0 |
| ZnO | | 0 bis 5,0 |
| F | | 0 bis 1,0 |
| Färbende und/oder fluoreszierende | | |
| Komponenten | | 0 bis 10,0, bevorzugt 0 bis 7,0, |

wobei die färbenden und/oder fluoreszierenden Komponenten insbesondere ausgewählt sind aus der Gruppe von Oxiden von Sn, Ce, V, Mn, Co, Ni, Cu, Fe, Cr, Tb, Eu, Er und Pr.

Die Erfindung ist ebenfalls auf einen mehrfarbigen Glaskeramik-Rohling gerichtet, der nach dem erfindungsgemäßen Verfahren erhältlich ist. Gegenüber Rohlingen, die durch die Massivglas-Technologie erhalten wurden, zeichnet sich der erfindungsgemäße Rohling nicht nur durch die deutlich einfachere Möglichkeit aus, Mehrfarbigkeit zu erzeugen, sondern er kann auch in kürzerer Zeit und mit geringerem Werkzeugverschleiß maschinell bearbeitet werden. Das ist bei der heutzutage gewünschten sehr schnellen und kostengünstigen Herstellung von hochästhetischen Dentalrestaurationen ein ganz besonders wichtiger Vorteil.

Aufgrund der geschilderten besonderen Eigenschaften des erfindungsgemäßen Rohlings eignet er sich insbesondere zum Einsatz in der Zahnheilkunde und insbesondere als Dentalmaterial und bevorzugt zur Herstellung von Dentalrestaurationen. Die Erfindung betrifft daher auch die Verwendung des Rohlings als Dentalmaterial und bevorzugt zur Herstellung von dentalen Restaurationen und insbesondere von Kronen, Abutments, Abutmentkronen, Inlays, Onlays, Veneers, Schalen, Brücken sowie Überwürfen.

Die Erfindung ist schließlich auch auf ein Verfahren zur Herstellung einer Dentalrestauration gerichtet, bei dem
(i) das beschriebene erfindungsgemäße Verfahren durchgeführt wird, um mehrfarbigen Glaskeramik-Rohling herzustellen,
(ii) dem mehrfarbigen Glaskeramik-Rohling durch maschinelle Bearbeitung die Form der Dentalrestauration gegeben wird, und
(iii) mindestens eine Wärmebehandlung bei einer Temperatur von mehr als 750°C, bevorzugt 800 bis 900°C, durchgeführt wird.

Die maschinelle Bearbeitung in der Stufe (ii) erfolgt üblicherweise durch materialabtragende Verfahren und insbesondere durch Fräsen und/oder Schleifen. Es ist bevorzugt, dass die maschinelle Bearbeitung mit Computer-gesteuerten Fräs- und/oder Schleifvorrichtungen erfolgt. Derartige Vorrichtungen sind dem Fachmann bekannt und auch marktüblich.

In einer bevorzugten Ausführungsform des Verfahrens bewirkt die Wärmebehandlung in Schritt (iii) die Bildung von Lithiumdisilikat als Hauptkristallphase. Glaskeramiken mit Lithiumdisilikat als Hauptkristallphase zeichnen sich durch hervorragende mechanische Eigenschaften aus, wie sie für ein Material gefordert werden, welches das natürliche Zahnmaterial ersetzen soll. In der durch die Wärmebehandlung erzeugten Glaskeramik sind die Kristalle und insbesondere die Lithiumdisilikat-Kristalle überraschenderweise sehr homogen verteilt, obwohl die Glaskeramik nicht unter Verwendung der sogenannten Massivglastechnologie, d.h. unter Verwendung von gegossenen monolithischen Glasblöcken, hergestellt worden ist.

Es liegt nach Durchführung der Stufe (iii) eine Dentalrestauration vor, die über sehr gute mechanische Eigenschaften und eine hohe chemische Stabilität verfüget. Darüber hinaus gestattet sie aufgrund ihrer Mehrfarbigkeit eine ausgezeichnete Nachahmung der optischen Eigenschaften von natürlichem Zahnmaterial, z.B. von Farbverläufen vom Dentin zur Schneide.

Es ist bevorzugt, dass die erhaltene Dentalrestauration eine biaxiale Biegefestigkeit σ_{B} von mindestens 300 MPa, insbesondere 360 bis 600 MPa, bestimmt nach ISO 6872:2008 (Kolben-auf-drei-Kugeln-Prüfung) und/oder ein Bruchzähigkeit K_{lc} von mindestens 2,0 MPa m^{1/2}, insbesondere 2,1 bis 2,5 MPa m^{1/2}, bestimmt nach ISO 6872:2008 (SEVNB-Methode) hat.

Schließlich kann die Dentalrestauration auch ohne wesentliche Schrumpfung aus den erfindungsgemäßen Glaskeramik-Rohlingen erzeugt werden. Das beruht insbesondere darauf, dass die erfindungsgemäßen Rohlinge eine hohe Dichte von insbesondere 2,4 bis 2,6, bevorzugt 2,44 bis 2,56, haben und demnach nur eine sehr geringe Porosität aufweisen. Sie unterscheiden sich darin von den üblicherweise mittels Pulvertechnologie hergestellten Glaskeramik-Rohlingen, die regelmäßig eine hohe Porosität haben. Durch Einsatz der erfindungsgemäßen Rohlinge können daher in besonders einfacher Weise Dentalrestaurationen mit genau den gewünschten Abmessungen hergestellt werden.

Die mittels des erfindungsgemäßen Verfahrens hergestellte Dentalrestauration ist bevorzugt ausgewählt aus der Gruppe von Kronen, Abutments, Inlays, Onlays, Veneers, Schalen, Brücken und Überwürfen.

Die Erfindung wird im Folgenden anhand von Beispielen näher erläutert.

### Beispiele

Die Beispiele erläutern insbesondere die Herstellung von mehrfarbigen Glaskeramik-Blöcken mit graduellem Verlauf von Farbe und Transluzenz und deren Verwendung zur Herstellung von Dentalrestaurationen.

### Beispiele 1 bis 9

### A. Herstellung Lithiumsilikat-Gläser

Es wurden zunächst neun unterschiedliche Lithiumsilikat-Gläser mit der in der Tabelle I angegebenen Zusammensetzung hergestellt, wobei die Gläser entweder zur Simulation des Zahnbeins (Dentin) oder der Zahnschneide (Schneide) dienten. Diesen Gläsern wurden die ebenfalls in der Tabelle I angegebenen Zusätze zugefügt.

Zur Herstellung dieser Gläser wurde zunächst ein Gemisch entsprechender Rohstoffe bei 1500°C für eine Dauer von 1,5 h erschmolzen, wobei das Erschmelzen sehr gut ohne Bildung von Blasen oder Schlieren möglich war. Durch Eingießen der erhaltenen Schmelze in Wasser wurde jeweils eine Glasfritte hergestellt.

### B. Herstellung einfarbiger Glaskeramik-Blöcke zur Eigenschaftsbestimmung

Diese Glasfritten wurden zunächst in einer Walzenmühle FM 2/2 (Merz Aufbereitungstechnik GmbH, Deutschland) auf eine Größe von < 3 mm vorzerkleinert und danach in einer Gegenstrahlmühle AFG 100 (Hosokawa Alpine AG) weiter auf eine Größe von 15 µm (d₅₀-Wert) zerkleinert, um jeweils Glaspulver zu erzeugen.

Die erhaltenen Glaspulver wurden unter Verwendung eines Sprühgranulierers GPCG 3.1 (Glatt GmbH Deutschland) granuliert, indem wässrige Suspensionen mit 1,0 Gew.-% Bindemittel und 0,5 Gew.-% Presshilfsmittel auf die Glaspulver in einer Wirbelschicht gesprüht wurden.

Die granulierten Glaspulver wurden dann in eine 3-teilige Stahlform bestehend aus Matrize sowie Ober- und Unterstempel eingebracht, um jeweils einen einfarbigen Glasrohling zu erzeugen.

Diese einfarbigen Glasrohlinge wurden in einer isostatischen Presse bei Raumtemperatur durch Pressen bei dem in Tabelle II angegebenen Druck verdichtet.

Die verdichteten Rohlinge wurden unter den in Tabelle II angegebenen Bedingungen in einem Sinterofen N11/HR (Nabertherm GmbH, Deutschland) entbindert und zu Glaskeramik-Rohlingen mit Lithiummetasilikat als Hauptkristallphase kristallisiert. Diese Glaskeramik-Rohlinge wurden anschließend in einer Drucksinterpresse DSP 515 (Dr. Fritsch Sondermaschinenbau GmbH, Deutschland) unter den ebenfalls in der Tabelle II angegebenen Bedingungen heißgepresst. Die Dichte der erhaltenen Glaskeramik-Rohlinge wurde nach dem Archimedes-Prinzip gemessen und ebenso wurde ihr Gehalt an Lithiummetasilikat durch Röntgenbeugungsuntersuchungen mit Rietveld-Analyse bestimmt. Die erhaltenen Werte sind in Tabelle II aufgeführt.

Schließlich wurden diese Rohlinge unter den ebenfalls in der Tabelle II angegebenen Bedingungen weiter kristallisiert, um Lithiumdisilikat als Hauptkristallphase zu erzeugen. Die auf diese Weise hergestellten Lithiumdisilikat-Rohlinge hatten die ebenfalls in der Tabelle II angegebenen Eigenschaften. Die biaxiale Biegefestigkeit σ_{B} wurde nach ISO 6872:2015 (Kolbenauf-drei-Kugeln-Prüfung) gemessen und die Bruchzähigkeit K_{lc} wurde nach ISO 6872:2015 (SEVNB-Methode) bestimmt. Zur Ermittlung der Lab-Werte sowie des Kontrastwertes (CR-Wert) wurde ein Spektralphotometer CM-3700d von Konica Minolta verwendet, wobei der Kontrastwert gemäß British Standard BS 5612 bestimmt wurde. Die Dichte wurde nach dem Archimedes-Prinzip bestimmt und der Gehalt an Lithiummetasilikat und Lithiumdisilikat wurde durch Röntgenbeugungsuntersuchungen mit Rietveld-Analyse gemessen.

Die Biegefestigkeit dieser durch Pulvertechnologie erzeugten Lithiumdisilikat-Rohlinge war überraschenderweise sehr hoch und vergleichbar mit derjenigen von Lithiumdisilikat-Glaskeramik, die in herkömmlicher Weise durch die Massivglas-Technologie erzeugt wurde und deren Festigkeit regelmäßig mindestens 360 MPa beträgt.

Auch die Bruchzähigkeit der Lithiumdisilikat-Rohlinge war erstaunlicherweise durchaus vergleichbar mit der von Lithiumdisilikat-Glaskeramik, die in herkömmlicher Weise durch die Massivglas-Technologie erzeugt wurde und typischerweise eine Bruchzähigkeit von 2,2 bis 2,3 MPam^{1/2} hat.

### C. Herstellung mehrfarbiger Glaskeramik-Blöcke

### a) Gradientenblöcke aus Glaspulvern gemäß Beispiel 1 und 2

Zur Erzeugung von mehrfarbigen Glaskeramik-Blöcken wurden granuliertes Glaspulver gemäß Beispiel 1 zur Simulation des Dentins und granuliertes Glaspulver gemäß Beispiel 2 zur Simulation der Schneide verwendet.

Diese granulierten Pulver wurden in der gleichen Weise hergestellt, wie es unter B. oben erläutert ist. Die Pulver wurden dann unter Verwendung eine Vorrichtung zum graduellen Dosieren und Mischen in der Weise in eine unter B. erwähnte dreiteilige Stahlform eingebracht, dass Glasrohlinge mit graduellem Farb- und Transluzenz-Verlauf erzeugt wurden. Anschließend wurden die Glasrohlinge zu Glaskeramik-Blöcken mit Lithiummetasilikat als Hauptkristallphase in der Weise umgewandelt, wie es oben unter B. erläutert ist.

Die erhaltenen mehrfarbigen Glaskeramik-Blöcke wurden in einer CAD/CAM-Einheit maschinell zu Kronen verarbeitet. Dazu wurden die Blöcke mit einem geeigneten Halter versehen, und anschließend wurde ihnen in einer Schleifeinheit inLab MC XL (Sirona Dental GmbH, Deutschland) die gewünschte Form gegeben. Für die Verarbeitung der Blöcke konnten die gleichen Schleifparameter wie für kommerzielle e.max CAD-Blöcke, Ivoclar Vivadent, Liechtenstein, verwendet werden.

Die maschinelle Bearbeitbarkeit der Glaskeramik-Blöcke wurde im Vergleich zu kommerziellen Glaskeramik-Blöcken des Typs e.max CAD LT, Ivoclar Vivadent AG, Liechtenstein, überprüft, die durch die Massivglas-Technologie erzeugt wurden. Ebenso wurde die Werkzeuglebensdauer getestet. Dazu wurde auf der Schleifeinheit inLab MC XL immer dieselbe Molarenkrone aus mit Haltern versehenen Blöcken gleicher Abmessungen heraus geschliffen und die Zeit vom Start bis zum Prozessende wurde bestimmt. Für die Werkzeuglebensdauer wurde die Anzahl der Kronen bestimmt, die erzeugt werden konnten, bis die Einheit auf die Notwendigkeit des ersten Werkzeugwechsels hinwies.

Es zeigte sich, dass die erfindungsgemäßen Glaskeramik-Blöcke den kommerziellen Blöcken überlegen sind, indem sie um mindestens 10 % schneller mechanisch bearbeitet werden konnten und die Werkzeuglebensdauer um mindestens 35 % länger war.

Diese günstigen Eigenschaften prädestinieren die erfindungsgemäßen Glaskeramik-Blöcke für eine sehr schnelle Versorgung von Patienten mit einer Dentalrestauration, die sowohl von ihren optischen Eigenschaften als auch ihren mechanischen Eigenschaften sehr hohe Ansprüche erfüllt.

### b) Gradientenblöcke aus Glaspulvern gemäß Beispiel 3 und 4

Es wurden mehrfarbige Glaskeramik-Blöcke in der gleichen Weise wie oben unter a) beschrieben hergestellt, wobei als einziger Unterschied zur Simulation des Dentins Glaspulver gemäß Beispiel 3 und zur Simulation der Schneide Glaspulver gemäß Beispiel 4 verwendet wurden.

Auch diese Glaskeramik-Blöcke waren kommerziellen Blöcken dadurch deutlich überlegen, dass sie schneller mechanisch bearbeitet werden konnten und die Werkzeuglebensdauer länger war.

Diese günstigen Eigenschaften zeigten auch Glaskeramik-Blöcke, die analog zu a) und b) hergestellt worden waren, bei denen aber wenigstens eines der eingesetzten Glaspulver durch ein anderes in der Tabelle I aufgeführtes Glaspulver ersetzt worden war.

### D. Wärmebehandlung zur Erzeugung von Dentalrestaurationen

Die unter C. erhaltenen maschinell bearbeiteten Blöcke wurden dann einer Wärmebehandlung bei 840 °C für eine Dauer von 7 min unterworfen. Anschließend wurden die erhaltenen Kronen langsam auf Raumtemperatur abgekühlt und eine Röntgenbeugungsuntersuchung ergab, dass sie Lithiumdisilikat als Hauptkristallphase aufwiesen.

Die erhaltenen Kronen hatten eine hohe Festigkeit. Außerdem wiesen sie einen graduellen Verlauf von Farbe und Transluzenz von Dentin zu Schneide auf und sie simulierten damit in ausgezeichneter Weise die optischen Eigenschaften von natürlichem Zahnmaterial.

Der Mittelwert für die Biegefestigkeit von 12 untersuchten Proben betrug für Lithiumdisilikat-Blöcke, die aus dem Gradientenblock gemäß a) (Pulver nach Beispielen 1 und 2) hergestellt worden waren, 403,82 MPa mit einer Standardabweichung von 55,16 MPa. Der Mittelwert für die Bruchzähigkeit von 6 untersuchten Proben dieser Lithiumdisilikat-Blöcke betrug 2,27 MPam^{1/2} mit einer Standardabweichung von 0,15 MPam^{1/2}.

Der Mittelwert für die Biegefestigkeit von 12 untersuchten Proben betrug für Lithiumdisilikat-Blöcke, die aus dem Gradientenblock gemäß b) (Pulver nach Beispielen 3 und 4) hergestellt worden waren, 470,57 MPa mit einer Standardabweichung von 100,66 MPa.

**Tabelle I**

| **Beispiel** | **1** | **2** | **3** | **4** | **5** | **6** | **7** | **8** | **9** |
|---|---|---|---|---|---|---|---|---|---|
| | | | | | | | | | |
| **Verwendung als** | Dentin | Schneide | Dentin | Schneide | Dentin | Dentin | Dentin | Schneide | Dentin |
| **Zusammensetzung des Glases** | Gew. % | Gew. % | Gew. % | Gew.-% | Gew. % | Gew. % | Gew. % | Gew. % | Gew. % |
| SiO₂ | 66,736 | 67,770 | 67,098 | 67,433 | 68,479 | 67,100 | 66,307 | 68,288 | 66,693 |
| K₂O | 4,222 | 4,190 | 4,211 | 4,169 | 4,297 | 4,211 | 4,147 | 4,222 | 4,123 |
| SrO | 2,861 | 2,870 | 2,864 | 2,856 | 2,923 | 2,864 | 2,825 | 2,892 | 2,824 |
| Li₂O | 14,706 | 14,530 | 14,644 | 14,458 | 14,946 | 14,645 | 14,412 | 14,641 | 14,299 |
| Al₂O₃ | 3,001 | 2,000 | 2, 651 | 1,990 | 2,705 | 2, 651 | 2,465 | 2,015 | 1,968 |
| P₂O₅ | 3,601 | 3,600 | 3,601 | 3,582 | 3,675 | 3,601 | 3,550 | 3,628 | 3,543 |
| MgO | - | 0,400 | 0,140 | 0,398 | 0,100 | 0,140 | 0,197 | 0,405 | 0,394 |
| ZrO₂ | - | 1,670 | 0,585 | 2,159 | 0,501 | 0,350 | 0,823 | 1,823 | 2,136 |
| ZnO | 1, 970 | 1,950 | 1, 963 | 1, 940 | 2,003 | 1, 963 | 1,932 | 1, 965 | 1, 919 |
| CeO₂ | 2,000 | 0,700 | 1,545 | 0,697 | 0,200 | 1,400 | 1,597 | 0,101 | 1,303 |
| MnO₂ | 0,100 | 0,020 | 0,072 | 0,020 | - | 0,025 | 0,158 | - | 0,099 |
| V₂O₅ | 0,150 | 0,100 | 0,133 | 0,100 | 0,010 | 0,250 | 0,399 | 0,010 | 0,401 |
| Tb₄O₇ | 0,500 | 0,200 | 0,395 | 0,199 | 0,150 | 0,500 | 0,838 | - | 0,197 |
| Er₂O₃ | 0,150 | - | 0,098 | - | 0,010 | 0,300 | 0,349 | 0,010 | 0,100 |
| Co₃O₄ | - | - | - | - | - | - | - | - | 0,001 |

| **Zusätze zu dem Glas** | | | | | | | | | |
|---|---|---|---|---|---|---|---|---|---|
| Fluoreszenzpigment | - | - | - | - | 1,00 | 2,00 | 5,00 | 1,00 | - |
| Additive | 1,50 | 1,50 | 1,50 | 1,50 | - | - | - | - | - |

| | | | | | | | | | |
|---|---|---|---|---|---|---|---|---|---|
| Mengen der Zusätze sind auf das Glas bezogen; Additive waren 1,0 Gew.-% Bindemittel und 0,5 Gew.-% Presshilfsmittel | | | | | | | | | |

**Tabelle II**

| **Beispiel** | **1** | **2** | **3** | **4** | **5** | **6** | **7** | **8** | **9** |
|---|---|---|---|---|---|---|---|---|---|
| | | | | | | | | | |
| Kaltpressen Druck (MPa) | 120 | 120 | 120 | 120 | 50 | 50 | 50 | 50 | 50 |
| Entbindern (min/°C) | 30/440 | 30/440 | 30/440 | 30/440 | - | - | - | - | - |
| Wärmebehandlung zur Kristallisation LS (min/°C) | 10/670 | 10/670 | 10/670 | 10/670 | 10/670 | 10/670 | 10/670 | 10/670 | 10/670 |
| Heißpressen (min/°C; MPa) | 10/750; 30 | 10/750; 30 | 10/750; 30 | 10/750; 30 | 5/750; 30 | 3/750; 30 | 5/750; 30 | 3/750; 30 | 5/750; 30 |
| Dichte LS-Rohling | 2,511 | 2,506 | - | 2,528 | - | - | - | - | - |
| LS-Gehalt LS-Rohling (%) | 34,7 | 33,2 | - | - | - | - | - | - | - |
| LS2-Gehalt LS-Rohling (%) | * | 1,6 | | | | | | | |
| | | | | | | | | | |
| Wärmebehandlung zur Kristallisation LS2 (min/°C) | 10/850 | 10/850 | 10/850 | 10/850 | 7/840 | 7/840 | 7/840 | 7/840 | 7/840 |
| Dichte LS2-Rohling | 2,533 | 2,524 | - | - | - | - | - | - | - |
| LS-Gehalt LS2-Rohling (%) | 8,0 | 3,7 | - | - | - | 6,3 | 6,2 | 3,2 | - |
| LS2-Gehalt LS2-Rohling (%) | 35,2 | 41,4 | - | - | - | 39,4 | 38,0 | 44,5 | - |
| | | | | | | | | | |
| L | 77,23 | 86,3 | - | - | 93,05 | 79,14 | 69,59 | 90,24 | 70,54 |
| a | 6,24 | -0,52 | - | - | 0,02 | 5,57 | 9,36 | 0,36 | 6,11 |
| b | 18,64 | 15,08 | - | - | 3,42 | 25,68 | 27,3 | 5,52 | 24,85 |
| CR | 94,11 | 65,77 | - | - | 70,02 | 78,25 | 83,7 | 61,97 | 81,75 |
| | | | | | | | | | |
| σ_{B} (MPa) | 447 | 403 | - | 471 | 392 | - | - | - | - |
| K_{lc} (MPa m^{1/2}) | 2,19 | 2,45 | - | - | - | - | - | - | - |

| | | | | | | | | | |
|---|---|---|---|---|---|---|---|---|---|
| LS - Lithiummetasilikat; LS2 - Lithiumdisilikat; * nicht nachweisbar | | | | | | | | | |

## Patentansprüche

1. Verfahren zur Herstellung eines mehrfarbigen Glaskeramik-Rohlings für dentale Zwecke mit Lithiumsilikat als Hauptkristallphase, bei dem
(a) (i) unterschiedlich gefärbte Pulver von Lithiumsilikat-Gläsern oder (ii) Suspensionen von unterschiedlich gefärbten Pulvern von Lithiumsilikat-Gläsern in flüssigen Medien in eine Form eingebracht werden, um einen Glas-Rohling zu bilden,
(b) gegebenenfalls der Glas-Rohling aus Schritt (a) durch Pressen verdichtet wird,
(c) der Glasrohling aus Schritt (a) oder (b) wärmebehandelt wird, um einen Glaskeramik-Rohling mit Lithiumsilikat als Hauptkristallphase zu erhalten und
(d) der Glaskeramik-Rohling aus Schritt (c) durch Heißpressen verdichtet wird.

2. Verfahren nach Anspruch 1, bei dem in Schritt (a) Pulver in die Form eingebracht werden und Schritt (b) durchgeführt wird.

3. Verfahren nach Anspruch 1, bei dem in Schritt (a) Suspensionen in die Form eingebracht werden und die flüssigen Medien abgetrennt werden.

4. Verfahren nach einem der Ansprüche 1 bis 3, bei dem in Schritt (a) die Pulver oder Suspensionen in der Weise in die Form eingebracht werden, dass der hergestellte mehrfarbige Glaskeramik-Rohling eine sich kontinuierlich verändernde Farbe hat.

5. Verfahren nach einem der Ansprüche 1 bis 4, bei dem in Schritt (a) die Pulver (i) eine Teilchengröße von 0,5 bis 150 µm, bevorzugt 1 bis 100 µm, und die Pulver der Suspensionen (ii) eine Teilchengröße von 0,5 bis 80 µm, bevorzugt 0,5 bis 70 µm, haben und/oder die Pulver (i) und die Pulver (ii) eine mittlere Teilchengröße als d₅₀-Wert von 5 bis 30 µm, bevorzugt 10 bis 20 µm, haben.

6. Verfahren nach einem der Ansprüche 1 bis 5, bei dem in Schritt (b) das Pressen bei einer Temperatur von weniger als 60°C, bevorzugt bei 15 bis 35°C, und insbesondere bei einem Druck von 20 bis 120 MPa, bevorzugt 50 bis 120 MPa, erfolgt.

7. Verfahren nach einem der Ansprüche 1 bis 6, bei dem in Schritt (c) die Wärmebehandlung bei einer Temperatur von weniger als 700°C, bevorzugt 550°C bis 690°C, und für eine Dauer von insbesondere 2 bis 60 min, bevorzugt 5 bis 30 min, erfolgt.

8. Verfahren nach einem der Ansprüche 1 bis 7, bei dem in Schritt (d) das Heißpressen bei einer Temperatur von 650 bis 780°C, insbesondere 700 bis 750°C, und bei einem Druck von insbesondere 5 bis 50 MPa, bevorzugt 10 bis 30 MPa, erfolgt.

9. Verfahren einem der Ansprüche 1 bis 8, bei dem in Schritt (d) das Heißpressen für eine Dauer von 0,1 bis 10 min, bevorzugt 0,3 bis 5 min, erfolgt.

10. Verfahren nach einem der Ansprüche 1 bis 9, bei dem in Schritt (d) das Heißpressen bei einem Atmosphärendruck von weniger als 0,1 bar und bevorzugt von 0,01 bis 0,08 bar erfolgt.

11. Verfahren nach einem der Ansprüche 1 bis 10, bei dem der mehrfarbige Glaskeramik-Rohling Lithiummetasilikat als Hauptkristallphase aufweist.

12. Verfahren nach einem der Ansprüche 1 bis 11, bei dem der mehrfarbige Glaskeramik-Rohling mehr als 10 Gew.-%, bevorzugt mehr als 20 Gew.-% und besonders bevorzugt mehr als 25 Gew.-% an Lithiummetasilikat-Kristallen enthält.

13. Verfahren nach einem der Ansprüche 1 bis 12, bei dem der mehrfarbige Glaskeramik-Rohling eine Dichte von 2,4 bis 2,6 und insbesondere 2,44 bis 2,56 g/cm³ hat.

14. Verfahren nach einem der Ansprüche 1 bis 13, bei dem der mehrfarbige Glaskeramik-Rohling mindestens eine und vorzugsweise alle der folgenden Komponenten in den angegebenen Mengen enthält:
| Komponente | | Gew.-% |
|---|---|---|
| | | |
| SiO₂ | | 64,0 bis 75,0 |
| Li₂O | | 13,0 bis 17,0 |
| K₂O | | 0 bis 5,0 |
| Al₂O₃ | | 0,5 bis 5,0 |
| P₂O₅ | | 2,0 bis 5,0 |

15. Verfahren nach einem der Ansprüche 1 bis 14, bei dem der mehrfarbige Glaskeramik-Rohling mindestens eine und vorzugsweise alle der folgenden Komponenten in den angegebenen Mengen enthält:
| Komponente | | Gew.-% | | |
|---|---|---|---|---|
| | | | | |
| SiO₂ | | 64,0 | bis | 75,0 |
| Li₂O | 13,0 | | bis | 17,0 |
| K₂O | | 0 | bis | 5,0 |
| Al₂O₃ | | 0,5 | bis | 5,0 |
| P₂O₅ | | 2,0 | bis | 5,0 |
| ZrO₂ | | 0 | bis | 5,0 |
| MgO | | 0 | bis | 5,0 |
| SrO | | 0 | bis | 5,0 |
| ZnO | | 0 | bis | 5,0 |
| F | | 0 | bis | 1,0 |
| Färbende und/oder fluoreszierende | | | | |
| Komponenten | | 0 | bis | 10,0, |
wobei die färbenden und/oder fluoreszierenden Komponenten insbesondere ausgewählt sind aus der Gruppe von Oxiden von Sn, Ce, V, Mn, Co, Ni, Cu, Fe, Cr, Tb, Eu, Er und Pr.

16. Mehrfarbiger Glaskeramik-Rohling erhältlich gemäß einem Verfahren nach einem der Ansprüche 1 bis 15.

17. Verwendung des mehrfarbigen Rohlings nach Anspruch 16 als Dentalmaterial und bevorzugt zur Herstellung von dentalen Restaurationen.

18. Verfahren zur Herstellung einer Dentalrestauration, bei dem
(i) das Verfahren nach einem der Ansprüche 1 bis 15 durchgeführt wird, um mehrfarbigen Glaskeramik-Rohling herzustellen,
(ii) dem mehrfarbigen Glaskeramik-Rohling durch maschinelle Bearbeitung die Form der Dentalrestauration gegeben wird, und
(iii) mindestens eine Wärmebehandlung bei einer Temperatur von mehr als 750°C, bevorzugt 800 bis 900°C, durchgeführt wird.

19. Verfahren nach Anspruch 18, bei dem in Schritt (iii) die Wärmebehandlung die Bildung von Lithiumdisilikat als Hauptkristallphase bewirkt.

20. Verfahren nach Anspruch 18 oder 19, bei dem in Schritt (ii) die maschinelle Bearbeitung mit computergesteuerten Fräs- und/oder Schleifvorrichtungen erfolgt.

21. Verfahren nach einem der Ansprüche 18 bis 20, bei dem die Dentalrestauration ausgewählt ist aus der Gruppe von Kronen, Abutments, Inlays, Onlays, Veneers, Schalen, Brücken und Überwürfen.

## Claims

1. Process for the preparation of a multi-coloured glass ceramic blank for dental purposes with lithium silicate as main crystal phase, in which
(a) (i) differently coloured powders of lithium silicate glasses or (ii) suspensions of differently coloured powders of lithium silicate glasses in liquid media are introduced into a mould to form a glass blank,
(b) optionally, the glass blank from step (a) is compacted by pressing,
(c) the glass blank from step (a) or (b) is heat treated to obtain a glass ceramic blank with lithium silicate as main crystal phase, and
(d) the glass ceramic blank from step (c) is compacted by hot pressing.

2. Process according to claim 1, in which in step (a) powders are introduced into the mould and step (b) is carried out.

3. Process according to claim 1, in which in step (a) suspensions are introduced into the mould and the liquid media are removed.

4. Process according to any one of claims 1 to 3, in which in step (a) the powders or suspensions are introduced into the mould in such a way that the multi-coloured glass ceramic blank prepared has a continuously changing colour.

5. Process according to any one of claims 1 to 4, in which in step (a) the powders (i) have a particle size of from 0.5 to 150 µm, preferably 1 to 100 µm, and the powders of the suspensions (ii) have a particle size of from 0.5 to 80 µm, preferably 0.5 to 70 µm, and/or the powders (i) and the powders (ii) have an average particle size as d₅₀ value of from 5 to 30 µm, preferably 10 to 20 µm.

6. Process according to any one of claims 1 to 5, in which in step (b) the pressing is effected at a temperature of less than 60°C, preferably at 15 to 35°C, and in particular at a pressure of from 20 to 120 MPa, preferably 50 to 120 MPa.

7. Process according to any one of claims 1 to 6, in which in step (c) the heat treatment is effected at a temperature of less than 700°C, preferably 550°C to 690°C, and for a period of in particular from 2 to 60 min, preferably 5 to 30 min.

8. Process according to any one of claims 1 to 7, in which in step (d) the hot pressing is effected at a temperature of from 650 to 780°C, in particular 700 to 750°C, and at a pressure of in particular from 5 to 50 MPa, preferably 10 to 30 MPa.

9. Process according to any one of claims 1 to 8, in which in step (d) the hot pressing is effected for a period of from 0.1 to 10 min, preferably 0.3 to 5 min.

10. Process according to any one of claims 1 to 9, in which in step (d) the hot pressing is effected at an atmospheric pressure of less than 0.1 bar and preferably of from 0.01 to 0.08 bar.

11. Process according to any one of claims 1 to 10, in which the multi-coloured glass ceramic blank has lithium metasilicate as main crystal phase.

12. Process according to any one of claims 1 to 11, in which the multi-coloured glass ceramic blank comprises more than 10 wt.-%, preferably more than 20 wt.-% and particularly preferably more than 25 wt.-% lithium metasilicate crystals.

13. Process according to any one of claims 1 to 12, in which the multi-coloured glass ceramic blank has a density of from 2.4 to 2.6 and in particular 2.44 to 2.56 g/cm³.

14. Process according to any one of claims 1 to 13, in which the multi-coloured glass ceramic blank comprises at least one and preferably all of the following components in the amounts indicated:
| Component | wt.-% | |
|---|---|---|
| | | |
| SiO₂ | 64.0 to 75.0 | |
| Li₂O | 13.0 to 17.0 | |
| K₂O | | 0 to 5.0 |
| Al₂O₃ | | 0.5 to 5.0 |
| P₂O₅ | | 2.0 to 5.0 |

15. Process according to any one of claims 1 to 14, in which the multi-coloured glass ceramic blank comprises at least one and preferably all of the following components in the amounts indicated:
| Component | wt.-% | |
|---|---|---|
| | | |
| SiO₂ | 64.0 to 75.0 | |
| Li₂O | 13.0 to 17.0 | |
| K₂O | | 0 to 5.0 |
| Al₂O₃ | | 0.5 to 5.0 |
| P₂O₅ | | 2.0 to 5.0 |
| ZrO₂ | | 0 to 5.0 |
| MgO | | 0 to 5.0 |
| SrO | | 0 to 5.0 |
| ZnO | | 0 to 5.0 |
| F | | 0 to 1.0 |
| colouring and/or | | |
| fluorescent components | | 0 to 10.0, |
wherein the colouring and/or fluorescent components are in particular selected from the group of oxides of Sn, Ce, V, Mn, Co, Ni, Cu, Fe, Cr, Tb, Eu, Er and Pr.

16. Multi-coloured glass ceramic blank obtainable according to a process according to any one of claims 1 to 15.

17. Use of the multi-coloured blank according to claim 16 as dental material and preferably for the preparation of dental restorations.

18. Process for the preparation of a dental restoration, in which
(i) the process according to any one of claims 1 to 15 is carried out to produce a multi-coloured glass ceramic blank,
(ii) the multi-coloured glass ceramic blank is given the shape of the dental restoration by machining, and
(iii) at least one heat treatment at a temperature of more than 750°C, preferably 800 to 900°C, is carried out.

19. Process according to claim 18, in which in step (iii) the heat treatment effects the formation of lithium disilicate as main crystal phase.

20. Process according to claim 18 or 19, in which in step (ii) the machining is effected with computer-controlled milling and/or grinding devices.

21. Process according to any one of claims 18 to 20, in which the dental restoration is selected from the group of crowns, abutments, inlays, onlays, veneers, facets, bridges and caps.

## Revendications

1. Procédé de fabrication d'une ébauche de pièce multicolore en vitrocéramique pour applications en dentisterie, comportant du silicate de lithium en tant que principale phase cristalline, dans lequel
a) on place dans un moule
i) des poudres de verres de silicate de lithium, de couleurs différentes,
ii) ou des suspensions, dans des milieux liquides, de poudres de verres de silicate de lithium, de couleurs différentes,
pour en faire une ébauche en verre,
b) en option, on comprime par pressage l'ébauche en verre issue de l'étape (a),
c) on soumet l'ébauche en verre issue de l'étape (a) ou (b) à un traitement thermique, pour obtenir une ébauche en vitrocéramique comportant du silicate de lithium en tant que principale phase cristalline,
d) et l'on comprime par pressage à chaud l'ébauche en vitrocéramique issue de l'étape (c).

2. Procédé conforme à la revendication 1, dans lequel ce sont des poudres qu'on place dans le moule, dans l'étape (a), et l'on effectue l'étape (b).

3. Procédé conforme à la revendication 1, dans lequel ce sont des suspensions qu'on place dans le moule, dans l'étape (a), et l'on en sépare les milieux liquides.

4. Procédé conforme à l'une des revendications 1 à 3, dans lequel, dans l'étape (a), on place les poudres ou les suspensions dans le moule de telle sorte que l'ébauche de pièce multicolore en vitrocéramique fabriquée présente une couleur qui varie continûment.

5. Procédé conforme à l'une des revendications 1 à 4, dans lequel, dans l'étape (a), les poudres (i) comportent des particules qui ont une taille de 0,5 à 150 µm, et de préférence de 1 à 100 µm, et les poudres des suspensions (ii) comportent des particules qui ont une taille de 0,5 à 80 µm, et de préférence de 0,5 à 70 µm, et/ou la taille moyenne, exprimée par la valeur de d₅₀, des particules des poudres (i) et des poudres (ii) vaut de 5 à 30 µm, et de préférence de 10 à 20 µm.

6. Procédé conforme à l'une des revendications 1 à 5, dans lequel, dans l'étape (b), le pressage a lieu à une température de moins de 60 °C, et de préférence de 15 à 35 °C, et en particulier, avec une pression de 20 à 120 MPa, et de préférence de 50 à 120 MPa.

7. Procédé conforme à l'une des revendications 1 à 6, dans lequel, dans l'étape (c), le traitement thermique est effectué à une température de moins de 700 °C, et de préférence de 550 à 690 °C, et durant un laps de temps de 2 à 60 minutes, et de préférence de 5 à 30 minutes.

8. Procédé conforme à l'une des revendications 1 à 7, dans lequel, dans l'étape (d), le pressage à chaud a lieu à une température de 650 à 780 °C, en particulier de 700 à 750 °C, et avec une pression valant en particulier de 5 à 50 MPa, et de préférence de 10 à 30 MPa.

9. Procédé conforme à l'une des revendications 1 à 8, dans lequel, dans l'étape (d), le pressage à chaud dure de 0,1 à 10 minutes, et de préférence de 0,3 à 5 minutes.

10. Procédé conforme à l'une des revendications 1 à 9, dans lequel, dans l'étape (d), le pressage à chaud a lieu sous une pression ambiante de moins de 0,1 bar, et de préférence de 0,01 à 0,08 bar.

11. Procédé conforme à l'une des revendications 1 à 10, dans lequel l'ébauche de pièce multicolore en vitrocéramique comporte, en tant que phase cristalline principale, du métasilicate de lithium.

12. Procédé conforme à l'une des revendications 1 à 11, dans lequel l'ébauche de pièce multicolore en vitrocéramique contient plus de 10 % en poids, de préférence plus de 20 % en poids, et mieux encore plus de 25 % en poids de cristaux de métasilicate de lithium.

13. Procédé conforme à l'une des revendications 1 à 12, dans lequel l'ébauche de pièce multicolore en vitrocéramique présente une masse volumique qui vaut de 2,4 à 2,6 g/cm³, et en particulier de 2,44 à 2,56 g/cm³.

14. Procédé conforme à l'une des revendications 1 à 13, dans lequel l'ébauche de pièce multicolore en vitrocéramique comporte au moins l'un des constituants suivants, mais de préférence, les comporte tous, en les proportions indiquées :
| Constituant | | % en poids |
|---|---|---|
| | SiO₂ | 64,0 à 75,0 |
| | Li₂O | 13,0 à 17,0 |
| | K₂O | 0 à 5,0 |
| | Al₂O₃ | 0,5 à 5,0 |
| | P₂O₅ | 2,0 à 5,0 |

15. Procédé conforme à l'une des revendications 1 à 14, dans lequel l'ébauche de pièce multicolore en vitrocéramique comporte au moins l'un des constituants suivants, mais de préférence, les comporte tous, en les proportions indiquées :
| Constituant | | % en poids |
|---|---|---|
| | SiO₂ | 64,0 à 75,0 |
| | Li₂O | 13,0 à 17,0 |
| | K₂O | 0 à 5,0 |
| | Al₂O₃ | 0,5 à 5,0 |
| | P₂O₅ | 2,0 à 5,0 |
| | ZrO₂ | 0 à 5,0 |
| | MgO | 0 à 5,0 |
| | SrO | 0 à 5,0 |
| | ZnO | 0 à 5,0 |
| | F | 0 à 1,0 |
| | Composants colorants | |
| | et/ou fluorescents | 0 à 10,0 |
étant entendu que ces composants colorants et/ou fluorescents sont choisis, en particulier, dans l'ensemble constitué par les oxydes des éléments Sn, Ce, V, Mn, Co, Ni, Cu, Fe, Cr, Tb, Eu, Er et Pr.

16. Ebauche de pièce multicolore en vitrocéramique, accessible par un procédé conforme à l'une des revendications 1 à 15.

17. Utilisation d'une ébauche de pièce multicolore, conforme à la revendication 16, en tant que matériau de dentisterie, et de préférence, pour la fabrication de pièces de restauration dentaire.

18. Procédé de fabrication d'une pièce de restauration dentaire, dans lequel
i) on met en œuvre un procédé conforme à l'une des revendications 1 à 15, pour fabriquer une ébauche de pièce multicolore en vitrocéramique,
ii) on donne à cette ébauche multicolore en vitrocéramique, par travail à la machine, la forme de la pièce de restauration dentaire,
iii) et on réalise au moins un traitement thermique à une température de plus de 750 °C, et de préférence de 800 à 900 °C.

19. Procédé conforme à la revendication 18, dans lequel le traitement thermique de l'étape (iii) provoque la formation de disilicate de lithium en tant que principale phase cristalline.

20. Procédé conforme à la revendication 18 ou 19, dans lequel le travail à la machine de l'étape (ii) est réalisé au moyen de dispositifs de fraisage et/ou de meulage commandés par ordinateur.

21. Procédé conforme à l'une des revendications 18 à 20, dans lequel la pièce de restauration dentaire est choisie dans l'ensemble formé par les couronnes, piliers, incrustations « in-lay » ou « on-lay », facettes, coquilles, bridges et superstructures.
